# EUROPÄISCHE PATENTANMELDUNG

(11) **EP 2 677 027 A1**
(43) Veröffentlichungstag der Anmeldung: **25.12.2013**
(21) Anmeldenummer: 12173056.8
(22) Anmeldetag: 21.06.2012
(51) Int. Cl.: C12N 5/071, A61L 27/38

(54) **Verfahren zur Herstellung von funktionalem Fusionsgewebe aus humanen Chondrozyten**

(71) Anmelder: Hochschule Lausitz, 01968 Senftenberg (DE)
(72) Erfinder: Anderer, Ursula, 01968 Senftenberg (DE); Lehmann, Mario, 01968 Großkoschen (DE); Martin, Frank, 01968 Senftenberg (DE)
(74) Vertreter: Simandi, Claus

(57) **Zusammenfassung**

Die vorliegende Erfindung betrifft ein Verfahren zur Herstellung von funktionalem Fusionsgewebe in einer dreidimensionalen gerüstfreien Fusionskultur, das mit diesem Verfahren erhältliche funktionale Fusionsgewebe sowie dessen Verwendung insbesondere als pharmazeutische Zubereitung, Arzneimittel, Transplantat, Testsystem.

## Beschreibung

Gegenstand der Erfindung ist ein Verfahren zur Herstellung von funktionalem Fusionsgewebe in einer dreidimensionalen gerüstfreien Fusionskultur, das mit diesem Verfahren erhältliche funktionale Fusionsgewebe sowie dessen Verwendung insbesondere als pharmazeutische Zubereitung, Arzneimittel, Transplantat und Testsystem.

Obwohl Gelenkknorpel eine bemerkenswerte Widerstandsfähigkeit zeigt, hat dieses Gewebe kein oder ein sehr geringes Vermögen zur Selbstreparatur und unbehandelte Läsionen können Osteoarthritis zur Folge haben. Dieses geringe Potential zur spontanen Regeneration führte zur Entwicklung von Zelltherapien, wie der autologen Chondrozytentransplantation (ACT), im Bestreben, eine funktionelle und schmerzlose Reparatur von Gelenkknorpeldefekten bereitzustellen (Brittberg et al., 1994). Derartige Techniken können jedoch auch nicht die Knorpelregeneration garantieren und es gibt noch immer nicht genug Langzeitstudien. Demzufolge besteht hoher Bedarf an Knorpelregeneration, im Einzelnen bei jungen aktiven Patienten mit traumatischen Läsionen oder sogar mit Symptomen einer Knorpeldegeneration. Zahlreiche Studien wurden mit Chondrozyten durchgeführt, die aus Knorpel von Rindern, Kaninchen oder Schafen isoliert wurden. Die erhaltenen Daten und derartige tierbasierte Konzepte sind jedoch bei weitem nicht auf die menschliche Situation übertragbar. Detaillierte biochemische und molekulare Studien mit humanen Chondrozyten wurden von einer Reihe von Faktoren behindert, wie der mangelnden Verfügbarkeit von Humangewebe in Verbindung mit der sehr geringen Anzahl von Zellen, die in einer Biopsie verfügbar sind, der begrenzten Proliferationskapazität und der hohen phänotypischen Instabilität kultivierter Chondrozyten.

Da ohne eine gegebene Gerüststruktur die Selbstorganisation eines Gewebes meist nicht gegeben ist, ist die Verwendung von Gerüsten (Scaffolds) oftmals entscheidend, wie beispielsweise beim Tissue Engineering von Blutgefäßen. Dabei ist die Auswahl eines passenden Gerüsts in der Praxis schwierig und oft nicht möglich.

Die DE 100 13 223 offenbart ein Verfahren zur in vitro Herstellung von dreidimensionalem Knorpel- und Knochengewebe aus Knochen-, Knorpel- oder mesenchymalen Stammzellen durch ein Verfahren, wobei die Zellen zunächst in einer Monolayerkultur und anschließend in Suspension so lange kultiviert werden, bis ein Zellaggregat entsteht, das zumindest 40 Vol % extrazelluläre Matrix beinhaltet, in welches differenzierte Zellen eingebettet sind und wobei die Kultivierung ohne wachstumsfördernde Verbindungen durchgeführt wird. DE 100 13 223 offenbart die Kultivierung in DMEM/Hams F12 Medium für mindestens 1-2 Wochen in Agarose beschichteten Zellkulturgefäßen. DE 100 13 223 offenbart auch, dass je nach gewünschter Gewebegröße mindestens zwei der entstandenen Zellaggregate fusioniert werden können, ohne jedoch konkrete Bedingungen für die Fusion zu nennen oder diese näher zu beschreiben. Die nach diesen Verfahren erzeugten Gewebe unterscheiden sich jedoch hinsichtlich ihrer Funktionalität und Expressionsmuster insbesondere im Hinblick auf die Expression von Kollagen Typ II jedoch deutlich von den entsprechenden nativen Geweben.

Es besteht deshalb weiterhin ein Bedürfnis an verbesserten in vitro erzeugten funktionalen Geweben.

Die Erfindung betrifft funktionale Fusionsgewebe, die nach einem neuen Verfahren hergestellt werden und die im Bezug auf ihre Funktionalität und Expressionsmuster insbesondere im Hinblick auf die Expression von Kollagen Typ II und spezifischen Proteoglykanen (z.B. knorpelspezifischen Proteoglykanen) denen des nativen Gewebes entsprechen bzw. weitgehend entsprechen.

Gegenstand der Erfindung ist ein Verfahren zur Herstellung von funktionalem Fusionsgewebe **dadurch gekennzeichnet, dass**
a) Zellen aus Gewebe humanen oder tierischen Ursprungs isoliert werden,
b) die isolierten Zellen in eine andere Umgebung gebracht und vermehrt werden,
c) aus den vermehrten Zellen Sphäroide (Zellaggregate, Mikrogewebe) hergestellt werden,
d) fünf oder mehr Sphäroide, gegebenenfalls in Gegenwart mindestens eines weiteren Differenzierungsinduktors, fusioniert werden.

Gegenstand der Erfindung ist auch ein Verfahren zur Herstellung von funktionalem Fusionsgewebe **dadurch gekennzeichnet, dass**
a) Zellen aus Gewebe humanen oder tierischen Ursprungs isoliert werden,
b) aus den isolierten Zellen dedifferenzierte Zellen hergestellt werden,
c) aus den dedifferenzierten Zellen Sphäroide (Zellaggregate, Mikrogewebe) hergestellt werden,
d) fünf oder mehr Sphäroide, gegebenenfalls in Gegenwart mindestens eines weiteren Differenzierungsinduktors, fusioniert werden.

Gegenstand der Erfindung ist ein Verfahren zur Herstellung von funktionalen Fusionsgeweben, nachfolgend auch "Fusionskultursystem" und "Fusionskultur" genannt, mit dem ohne weitere Hilfen wie Gerüste funktionales Fusionsgewebe hergestellt werden kann. Das mit diesem Verfahren hergestellte funktionale Fusionsgewebe besteht dabei aus Fusionen, d.h. mehreren miteinander fusionierten Sphäroiden. Das mit Hilfe des erfindungsgemäßen Verfahrens hergestellte Fusionsgewebe liegt vorzugsweise in Form von Mikrogewebe vor.

Funktionales Fusionsgewebe bedeutet, dass das Gewebe weitgehend dem nativen Gewebe entspricht. Im Sinne dieser Erfindung bedeutet das, dass das funktionale Fusionsgewebe beispielsweise in Bezug auf die Expression bzw. Expressionsmuster (z.B. immunhistologisch, histologisch) der extrazellulären Matrix weitgehend dem des nativen Gewebes entspricht. Das funktionale Fusionsgewebe entspricht beispielsweise in Bezug auf die Expression bzw. Expressionsmuster von Kollagen, insbesondere Kollagen Typ II und/oder Kollagen Typ I und/oder S100 Protein und/oder gewebespezifische Proteoglykane, z.B. knorpelspezifische Proteoglykane (bei funktionalem Knorpelgewebe, beispielsweise Gelenkknorpelgewebe) dem des nativen Gewebes.

Natives Gelenkknorpelgewebe hat dabei eine anteilmäßige Zusammensetzung der extrazellulären Matrix von etwa 60 bis 80% Wasser im Bezug auf das Feuchtgewichts des Knorpelgewebes. Der hohe Wasseranteil ist zusammen mit den Proteoglykanen und Kollagenen wichtig für die mechanische Belastbarkeit des Knorpelgewebes. Neben Wasser enthält das native Knorpelgewebe die strukturellen Makromoleküle der Matrix, also Kollagene, Proteoglykane und nicht-Kollagen Proteine. Dabei machen die strukturellen Makromoleküle etwa 20 bis 40% des Feuchtgewichts des Gelenkknorpelgewebes aus.

Das funktionale Fusionsgewebe hat beispielsweise einen Wasseranteil von 50 bis 90 %, vorzugsweise 55 bis 85 % oder 60 bis 80 %, besonders bevorzugt 65 bis 75 % oder 70 % bezogen auf das Feuchtgewicht des funktionalen Fusionsgewebes. Das funktionale Fusionsgewebe hat beispielsweise 10 bis 50 % strukturelle Makromoleküle der Matrix, vorzugsweise 15 bis 45 % oder 20 bis 40 %, besonders bevorzugt 25 bis 35 % oder 30 % bezogen auf das Feuchtgewicht des funktionalen Gewebes. Dabei addieren sich die jeweiligen Wasseranteile, strukturellen Makromoleküle und gegebenenfalls weiteren Bestandteile zu 100 %.

Bei nativem Gelenkknorpelgewebe beträgt der Kollagenanteil 60%, der Proteoglykananteil 25 bis 35% und der nicht-Kollagenprotein- und Glycoproteinanteil 15 bis 20% in Bezug auf das Knorpeltrockengewicht. Das Hauptkollagen ist dabei Kollagen Typ II; es macht etwa 90 bis 95% des Gesamtkollagengehalts des nativen Gelenkknorpelgewebes aus.

Funktionales Fusionsgewebe enthält beispielsweise 50 bis 70 %, vorzugsweise 55 bis 65 %, besonders bevorzugt 60 % Kollagen im Bezug auf das Trockengewicht des funktionalen Fusionsgewebes. Funktionales Fusionsgewebe enthält beispielsweise 15 bis 45 %, vorzugsweise 20 bis 40 % besonders bevorzugt 25 bis 35 % Proteoglykane im Bezug auf das Trockengewicht des funktionalen Fusionsgewebes. Funktionales Fusionsgewebe enthält beispielsweise 60 bis 90 % oder mehr, vorzugsweise 85 bis 98 % oder 80 bis 98 %, besonders bevorzugt 85 bis 97 % oder 90 bis 96 % oder 95 % Kollagen Typ II bezogen auf den Gesamtkollagengehalt des funktionalen Fusionsgewebes.

Natives Gelenkknorpelgewebe des Menschen enthält etwa 1 bis 5% Knorpelzellen (Chondrozyten) im Bezug auf das Gewebevolumen. Die Knorpelzellen sind dabei die essentiellen Produzenten der Matrixmoleküle des funktionalen nativen Gewebes.

Funktionales Fusionsgewebe enthält beispielsweise 0,1 bis 10 % oder 0,2 bis 8 %, vorzugsweise 0,4 bis 6 % oder 0,5 bis 5 %, besonders bevorzugt 0,7 bis 3 % oder 0,9 bis 1 % Zellen, die die extrazellulären Matrixmoleküle produzieren wie beispielsweise Kollagen Typ II und Proteoglykane. Funktionales Fusionsgewebe kann aber auch bis zu 30% bis 50% Zellen enthalten, die die extrazellulären Matrixmoleküle produzieren wie beispielsweise Kollagen Typ II und Proteoglykane, beispielsweise 40 % oder 35 %, vorzugsweise 30 % oder 25 %, besonders bevorzugt 20 % oder 15 % oder weniger Zellen.

Eine Ausführungsform der Erfindung betrifft ein erfindungsgemäßes Verfahren, **dadurch gekennzeichnet, dass** 5 bis 10 oder mehr, vorzugsweise 6 oder 7, besonders bevorzugt 8 oder 9 Sphäroide fusioniert werden. In einer besonders bevorzugten Ausführungsform des Verfahrens werden 5 Sphäroide zur Herstellung des funktionalen Fusionsgewebes fusioniert.

Mit dem Verfahren kann funktionales Fusionsgewebe unterschiedlicher Größe hergestellt werden. Einzelne Sphäroide haben beispielsweise einen Durchmesser von ca. 700 - 1500 µm, vorzugsweise 800 - 1400 µm, besonders bevorzugt 1000 - 1300 µm. Entsprechend der Anzahl der fusionierten Sphäroide ergibt sich daraus auch die Größe des funktionalen Fusionsgewebes. Die Größe des funktionalen Fusionsgewebes hängt neben der Anzahl auch von der Selbstanordnung (Form) der fusionierten Einzelsphäroide ab. Im bevorzugten Fall von 5 Einzelsphäroiden in der Fusion könnte man die Maße in etwa mit 2000 - 3000 µm x 3000 - 4000 µm angeben. Bei mehr als 5 Einzelsphäroiden sind die Maße entsprechend größer. Die Größe kann beispielsweise mikroskopisch bestimmt werden. Die entsprechenden Methoden sind dem Fachmann bekannt.

Dabei ist die spezielle Anordnung der Zellen an der Oberfläche von Einzelsphäroiden in Schritt d) des Verfahrens entscheidend sowie das Vorhandensein mindestens eines Differenzierungsinduktors zur Herstellung von funktionalem Fusionsgewebe. Die enge räumliche Anordnung von mehreren Sphäroiden führt dabei nicht nur zur Koaleszenz (Verschmelzung) mehrerer kleinerer Zellaggregate (Sphäroide) sondern induziert auch die Redifferenzierung der dedifferenzierten Zellen in den Sphäroiden und dem daraus gebildeten Fusionsgewebe. Die enge räumliche Anordnung von 5 oder mehr Sphäroiden stellt einen Differenzierungsinduktor dar.

Ein erfindungswesentliches Merkmal ist deshalb die besondere 3D-Umgebung der Sphäroide bei der Herstellung des funktionalen Fusionsgewebes. "3D-Umgebung" bedeutet, dass eine einzelne Zelle in jeder Richtung mit einer anderen Zelle Kontakt aufnehmen kann. Durch diese Kontaktaufnahme der Zellen in allen drei Raumrichtungen entstehen Sphäroide mit einer kugelartigen Struktur. Die kugelartigen Sphäroide werden wiederum in einer "3D-Umgebung" auf engem Raum in Kontakt gebracht. Durch die dadurch mögliche Kontaktaufnahme der Zellen im Randbereich der einzelnen Sphäroide mit den Zellen im Randbereich der anderen Sphäroide bilden sich Fusionsgewebe, d.h. es können größere Gewebestücke hergestellt werden. Werden 5 oder mehr Sphäroide in einer 3D-Umgebung fusioniert, dann wird gleichzeitig die Bildung von funktionalem Fusionsgewebe induziert.

Die durch das erfindungsgemäße Verfahren erhaltenen Fusionsgewebe sind deshalb im Vergleich zu den im Stand der Technik, insbesondere in DE 100 13 223 beschriebenen, nicht nur größer, sondern auch in ihrer Funktionalität verschieden. Die erfindungsgemäßen Fusionsgewebe sind aus redifferenzierten Zellen aufgebaut, sie sind funktional und in ihrer Struktur den nativen Geweben, aus denen die verwendeten Zellen isoliert wurden, ähnlicher bzw. sehr ähnlich bzw. identisch.

Das erfindungsgemäße Verfahren wird vorzugsweise in vitro durchgeführt, um in vitro Mikrogewebe (funktionales Fusionsgewebe) herzustellen.

Gegenstand der Erfindung ist deshalb ein Verfahren zur Herstellung von funktionalem Fusionsgewebe unter Verwendung eines speziellen Kultursystems zusammen mit Faktoren, die die Differenzierung fördern, aber ohne Zuhilfenahme von Gerüstmaterial. Das Verfahren umfasst zwei Aggregationsschritte: Im ersten Aggregationsschritt werden aus den isolierten Zellen, die teilweise oder ganz dedifferenziert sein können, einzelne isolierte Sphäroide hergestellt (Verfahrensschritt c)), die dann im zweiten Aggregationsschritt (Verfahrensschritt d)) der Fusionskultivierung in Anwesenheit von Komponenten, die für die Zellkondensation und Zellkommunikation relevant sind (im Rahmen dieser Erfindung "Differenzierungsinduktor" genannt) weiter fusioniert werden.

Erfindungsgemäß werden im zweiten Aggregationsschritt mehrere Schichten der Oberflächen von in-vitro-Geweben, insbesondere von Sphäroiden in engem Kontakt zueinander angeordnet (erfindungsgemäß "Kultivierung in 3D-Umgebung") und dadurch die mesenchymale Kondensation nachgeahmt. Der Differenzierungsinduktor ist dabei die Zell-Zell- und/oder Zell-Matrix-Wechselwirkung und / oder die Ausbildung von Gap Junctions.

Erfindungsgemäß umfasst ist sowohl die Kombination von mehreren einzelnen Sphäroiden mit bereits fusionierten Geweben oder die Kombination von mehreren einzelnen Sphäroiden.

Eine weitere Ausführungsform der Erfindung betrifft ein erfindungsgemäßes Verfahren, **dadurch gekennzeichnet, dass** im Verfahrensschritt d) mindestens 1 x 10⁵ Zellen / Well einer Mikrotiterplatte, vorzugsweise 3 x 10⁵ Zellen / Well, besonders bevorzugt 5 x 10⁵ Zellen / Well oder mehr fusioniert werden.

"Differenzierungsinduktoren" im Sinne dieses Verfahrens sind vorzugsweise mechanische und/oder chemische und/oder biochemische Differenzierungsinduktoren. Als mechanische Differenzierungsinduktoren sind beispielsweise die Kultivierung in 3D-Umgebung mit mindestens 5 Sphäroiden, die Kultivierung in Gegenwart von mechanischen Stimuli wie Druckapplikation, Kultivierung in Bioreaktoren wie Roting Wall Vessel oder Spinner Flaschen geeignet. Als chemische Differenzierungsinduktoren sind beispielsweise Ascorbinsäure, insbesondere L-Ascorbinsäure und Derivate von Ascorbinsäure wie Ascorbat-2-phosphat geeignet. Als biochemische Differenzierungsinduktoren sind beispielsweise Proteine wie Proteine der TGF-ß Superfamilie beispielsweise die TGF-ß Isoformen (TGF-β1, TGF-β2, TGF-β3) und Bone Morphogenetic Proteins (BMP-2, BMP-3, BMP-4, BMP-5, BMP-6, BMP-7), Growth Differentiation Factor wie beispielsweise GDF-5 und GDF-10, Insulin-like growth factor wie IGF-1 geeignet. Grundsätzlich sind alle differenzierungsmodulierenden Substanzen als Differenzierungsinduktoren geeignet, beispielsweise auch Glucocorticoide wie Dexamethason. Differenzierungsinduktoren im Sinne der vorliegenden Erfindung sind alle Komponenten und Faktoren, die für die Zellkondensation und Zellkommunikation relevant sind oder die Zellkondensation und Zellkommunikation beeinflussen. Erfindungsgemäß können ein oder mehrere Differenzierungsinduktoren in dem Verfahren eingesetzt werden. Die Brauchbarkeit einzelner Differenzierungsinduktoren sowie die Eignung von Kombinationen von mehreren Differenzierungsinduktoren kann vom Fachmann beispielsweise anhand der Expressionsmuster von Kollagen Typ II, gewebespezifischen Proteoglykanen, Kollagen Typ I und/oder S100 Protein überprüft werden.

Eine bevorzugte Ausführungsform der Erfindung betrifft ein erfindungsgemäßes Verfahren, **dadurch gekennzeichnet, dass** der/die weiteren Differenzierungsinduktor(en) ausgewählt werden aus den Differenzierungsinduktoren TGF-ß zusammen mit Ascorbinsäure, insbesondere TGF-ß2 zusammen mit L-Ascorbinsäure.

Eine weitere Ausführungsform der Erfindung betrifft ein erfindungsgemäßes Verfahren, **dadurch gekennzeichnet, dass** das solide Gewebe, aus dem die Zellen isoliert werden, humanen oder tierischen Ursprungs ist. Das solide Gewebe kann ento-, ekto- und/oder mesodermalen Ursprungs sein. In besonderen Ausführungsformen der Erfindung ist das solide Gewebe ausgewählt aus muskoskeletalem Gewebe, Skelettgewebe, Knorpel, Knochen, Muskeln, glatter Muskulatur, Herzmuskel, Nieren, Nebennierenrinde, Bindegewebe, Meniskus, Leber. Im Rahmen der Erfindung werden vorzugsweise humane Zellen verwendet. Die Zellen sind vorzugsweise adulte Zellen. Die Zellen sind beispielsweise im Zellzyklus arretierte Zellen. Vorzugsweise werden frisch isolierte Zellen verwendet. In einer bevorzugten Ausführungsform der Erfindung werden adulte humane Chondrozyten, insbesondere frisch isolierte postmitotische Chondrozyten (Knorpelzellen) verwendet. In einer weiteren bevorzugten Ausführungsform der Erfindung werden adulte humane Knochenzellen, insbesondere Osteozyten und/oder Osteoblasten verwendet.

Eine weitere Ausführungsform der Erfindung betrifft ein erfindungsgemäßes Verfahren, **dadurch gekennzeichnet, dass** die Zellen Chondrozyten sind.

Eine weitere Ausführungsform der Erfindung betrifft ein erfindungsgemäßes Verfahren, **dadurch gekennzeichnet, dass** die Zellen tierische oder humane Zellen sind, beispielsweise frisch isolierte und/oder postmitotische Zellen.

Eine weitere Ausführungsform der Erfindung betrifft ein erfindungsgemäßes Verfahren, **dadurch gekennzeichnet, dass** die dedifferenzierten Zellen durch Kultur in Monolayer hergestellt werden.

Verfahrensschritt b) dient zur Vermehrung der isolierten Zellen. Die isolierten Zellen werden dazu in eine neue Umgebung gebracht. Durch das Einbringen von isolierten Zellen in die neue Umgebung kommt es zwangsläufig zur Dedifferenzierung der isolierten Zellen. Durch die Dedifferenzierung verändert sich die Funktionalität der Zellen, was beispielsweise durch die veränderten Expressionsmuster von Kollagen Typ II, Kollagen Typ I, gewebespezifischen Proteoglykanen und/oder S100 Protein nachgewiesen werden kann. Eine Ausführungsform der Erfindung betrifft ein erfindungsgemäßes Verfahren, wobei die isolierten Zellen dadurch vermehrt werden, dass sie in Monolayerkultur kultiviert werden, beispielsweise für zwei oder mehr Passagen.

Gegenstand der Erfindung ist ein Verfahren zur Herstellung von funktionalem Fusionsgewebe umfassend die Schritte
a) Expansion isolierter Zellen, insbesondere von Chondrozyten in einer Monolayerkultur unter Dedifferenzierung der Zellen,
b) Aggregation der isolierten und dedifferenzierten Zellen zu Sphäroiden,
c) Aggregation der Sphäroide unter Redifferenzierung der Zellen durch Kultivierung in einer 3D-Umgebung (Fusionskultivierung) ggf. in Gegenwart weiterer Differenzierungsinduktoren wie beispielsweise TGF-β2 und ggf. L-Ascorbinsäure.

Eine weitere Ausführungsform der Erfindung betrifft ein erfindungsgemäßes Verfahren, **dadurch gekennzeichnet, dass** das Verfahren einen weiteren Schritt umfasst, in dem die Bildung von Sphäroiden angeregt wird. Dazu werden die isolierten bzw. vermehrten bzw. dedifferenzierten Zellen in eine Umgebung gebracht in der sich die Zellen bevorzugt an andere Zellen und nicht an die Oberfläche der Umgebung anheften. Dazu können beispielsweise Gefäße mit einer hydrophoben Oberfläche für die Kultivierung verwendet werden. Eine weitere Ausführungsform der Erfindung betrifft deshalb ein Verfahren, wobei dedifferenzierten Zellen auf einer hydrophoben Oberfläche, vorzugsweise auf einer Agaroseschicht, kultiviert werden. Die Kultivierung auf Agarose regt die Sphäroidbildung an (Anderer und Libera, 2002).

Eine weitere Ausführungsform der Erfindung betrifft ein erfindungsgemäßes Verfahren, **dadurch gekennzeichnet, dass** die dedifferenzierten Zellen für 1 bis 5 Tage, vorzugsweise zwei Tage auf Agarose oder einer anderen hydrophoben Oberfläche kultiviert werden.

Für die Kultivierung werden erfindungsgemäß übliche Kulturmedien vorzugsweise ohne Zusatz von Antibiotika oder Fungistatika verwendet. Dem Kulturmedium wird vorzugsweise noch Serum zugesetzt, beispielsweise Humanserum in einer Konzentration von etwa 1 % bis 20 %, vorzugsweise 5 bis 10 %. Erfindungsgemäß geeignet sind alle üblichen Kulturmediem, beispielsweise HAMs, Alpha-Medium, DMEM, MEM. Eine besondere Ausführungsform des erfindungsgemäßen Verfahrens betrifft die Verwendung von Alpha-Medium und HAMs F12 (1:1) als Kulturmedium, wobei vorzugsweise L-Glutamin und Humanserum zugesetzt werden.

Die Fusion gemäß Schritt d) des erfindungsgemäßen Verfahrens erfolgt beispielsweise für 2 bis 8 Wochen, vorzugsweise 6 Wochen. Die Fusion gemäß Schritt d) des Verfahrens wird in einer besonderen Ausführungsform mit 5 Sphäroiden / Well einer Mikrotiterplatte und bevorzugt in Gegenwart von TGF-ß2 und ggf. L- Ascorbinsäure durchgeführt.

Gegenstand der Erfindung ist auch funktionales Fusionsgewebe erhältlich durch ein erfindungsgemäßes Verfahren. Beispielsweise umfasst die Erfindung nach dem Verfahren hergestelltes Fusionsgewebe, insbesondere Knorpel- und Knochengewebe, das ein autologes, xenogenes oder allogenes Fusionsgewebe im Hinblick auf den betreffenden Spender, Mensch oder Tier, des für das Verfahren eingesetzten Gewebes bzw. der eingesetzten Zellen ist.

Gegenstand der Erfindung ist auch die Verwendung von funktionalem Fusionsgewebe das nach dem erfindungsgemäßen Verfahren erhältlich ist, beispielsweise als Transplantat, funktionales Ersatzgewebe, zur in vitro Gewebekultivierung, zur Herstellung von größeren transplantierbaren Geweben, zur Herstellung von in vitro Geweben, zum Tissue Engineering. Insbesondere betrifft die Erfindung die Verwendung bzw. spezifische Anwendung des erfindungsgemäßen Fusionsgewebes als autologes, xenogenes oder allogenes Transplantat.

Gegenstand der Erfindung ist ein Transplantat/funktionales Ersatzgewebe erhältlich nach dem erfindungsgemäßen Verfahren. Das in vitro hergestellte Transplantat/funktionale Ersatzgewebe kann in das umliegende native Gewebe des Empfängers eingebracht werden. In dem nach dem erfindungsgemäßen Verfahren hergestellten funktionalen Fusionsgewebe, d.h. auch in dem Transplantat/funktionalen Ersatzgewebe findet keine Zellteilung statt. Wird das funktionale Fusionsgewebe in natives Gewebe eingebracht, dann heften sich die Zellen des funktionalen Fusionsgewebes an das native Gewebe an (Adhäsion) und wandern in die Lücken ein (Migration) ohne sich jedoch zu teilen. Dadurch ist eine optimale Versorgung/Widerherstellung gewährleistet, jedoch ohne das Risiko einer unkontrollierten Proliferation des funktionalen Ersatzgewebes.

Gegenstand der Erfindung ist auch die Verwendung / spezifische Anwendung des erfindungsgemäßen funktionalen Fusionsgewebes zusammen mit Sphäroiden.

Gegenstand der Erfindung ist auch eine Zubereitung, insbesondere eine pharmazeutische Zubereitung, ein Arzneimittel, ein Transplantat bestehend aus oder enthaltend funktionales Fusionsgewebe, dass nach dem erfindungsgemäßen Verfahren erhältlich ist und gegebenenfalls weitere Hilfs- und Zusatzstoffe.

Gegenstand der Erfindung sind auch pharmazeutische Zubereitungen und Arzneimittel, insbesondere Suspensionen und Lösungen, insbesondere Injektionslösungen, die das erfindungsgemäße funktionale Fusionsgewebe und ggf. weitere Hilfs- und Zusatzstoffe enthalten.

Gegenstand der Erfindung ist auch die spezifische therapeutische/pharmazeutische Anwendung einer erfindungsgemäßen pharmazeutischen Zubereitung, eines erfindungsgemäßen Arzneimittels, eines erfindungsgemäßen Transplantats zur Behandlung von Knorpeldefekten und / oder Knochendefekten, insbesondere traumatischen Knorpeldefekten und / oder Knochendefekten, Läsionen, insbesondere traumatischen Läsionen, bei Knorpeldegenerationen, Knochendegenerationen, Osteoarthritis, zur therapeutischen Knorpel- und / oder Knochenregeneration in vivo.

Gegenstand der Erfindung ist auch die Verwendung des erfindungsgemäßen Fusionsgewebes zum Testen von Wirkstoffen, beispielsweise um neue Wirkstoffe zu Screenen, bekannte Wirkstoffe zu verbessern oder validieren oder um für einen bekannten Wirkstoff neue Indikationen und Anwendungsgebiete zur erschließen oder im Rahmen der vorklinischen und klinischen Prüfung zur Datengenerierung und Prüfung von Wirkstoffen.

Gegenstand der Erfindung ist ein Testsystem, beispielsweise ein Test Kit umfassend
a) funktionales Fusionsgewebe welches durch das erfindungsgemäße Verfahren erhältlich ist,
b) gegebenenfalls weitere Hilfs- und Zusatzstoffe.

Gegenstand der Erfindung ist ein Testsystem umfassend funktionales Fusionsgewebe und gegebenenfalls weitere Zusatz- und/oder Hilfsstoffe. Gegenstand der Erfindung ist beispielsweise auch ein in vitro Testsystem für die experimentelle Pharmakologie. Mit einem solchen Testsystem können neue Wirkstoffe identifiziert und neue und bekannte Wirkstoffe getestet werden, beispielsweise der Effekt eines Arzneimittels auf Knochen und/oder Knorpel. Desweiteren kann der Effekt von Substanzen, künstlichen oder natürlichen Ursprungs auf beispielsweise Knochen und/oder Knorpel getestet werden, beispielsweise von Lebensmitteln, Naturstoffen, Lösungsmitteln, Polymeren etc.. Ein solches Testsystem kann damit beispielsweise Aufschluß über die Toxizität und mögliche Nebenwirkungen liefern und auch zur Bestimmung von Grenzwerten und synergistischen Effekten von Substanzen verwendet werden.

Gegenstand der Erfindung ist deshalb auch ein Verfahren zum Testen von zu untersuchenden Substanzen umfassend die Schritte
a) Herstellung von funktionalem Fusionsgewebe oder eines Testsystems, das funktionales Fusionsgewebe umfasst;
b) das Inkontakt bringen einer zu testenden Substanz mit dem funktionalen Fusionsgeweben aus a),
c) Bestimmung/Nachweis des Effekts der zu untersuchenden Substanz auf das funktionale Fusionsgewebe.

Der Nachweis der Dedifferenzierung und Redifferenzierung kann über S100, Kollagen Typ II, Kollagen Typ I und gewebespezifische Proteoglykane erfolgen. Die Dedifferenzierung der Zellen zeichnet sich dadurch aus, dass S100, Kollagen Typ II, gewebespezifische Proteoglykane vermindert exprimiert werden und Kollagen Typ I verstärkt exprimiert wird. Die Differenzierung nativer Zellen und die Differenzierung funktionalen Fusionsgewebes zeichnen sich durch eine hohe Expression von Kollagen Typ II, gewebespezifischen Proteoglykanen, S100 Protein aus und durch eine niedrige Expression von Kollagen Typ I.

Mesenchymale Stammzellen (MCS) besitzen ein hohes Proliferations- und Differenzierungspotential. Adulte Mesenchymale Stammzellen tragen zur Aufrechterhaltung und Regeneration des Stütz- und Bindegewebes, wie Knochen, Knorpel, Muskel, Bändern, Sehnen und Fettgewebe bei. Darüber hinaus unterstützen sie Wachstum und Entwicklung der Vorläuferzellen des Blutes im Knochenmark. MSC aus unterschiedlichen Geweben (Knochenmark, Knorpel, Fettgewebe, Muskel, Lebergewebe, Blut, Amnionflüssigkeit) lassen sich kultivieren und in vitro in unterschiedliche Gewebe ausdifferenzieren.

Ein Chondrozyt (Knorpelzelle), ist eine aus Chondroblasten hervorgehende und im Knorpelgewebe ansässige Zelle. Zusammen mit den Interzellularsubstanzen (extrazellulärer Matrix), bilden die Chondrozyten die Hauptbestandteile des Knorpels.

Humane Zellen nicht nur als zweidimensionale Einschichtkultur (Monolayer) sondern auch als dreidimensionale Zellaggregate, z.B. sogenannte Sphäroide, zu kultivieren ist bereits beschrieben sowie zur klinischen Anwendung am Menschen, z.B. als autologes Knorpeltransplantat, zugelassen (DE 100 13 223).

Das Verfahren der Fusionskultur ist dagegen eine Neuentwicklung, die es ermöglicht einzelne Zellaggregate zu einem größeren "knorpelähnlichen" Gewebe zu vereinen, welches dann auch wesentlich verbesserte Eigenschaften hinsichtlich der Differenzierung und der Gewebequalität aufweist, was schließlich eine Anwendung als in vitro Testsystem für die pharmazeutische Industrie oder als Transplantat in der regenerativen Medizin begünstigt.

Es ist bereits bekannt, durch Mimikry bestimmter Prozesse der Embryonalentwicklung, z.B. menschliche Knorpelzellen dreidimensional auf einer Agaroseunterlage zu kultivieren, so dass Zellaggregate entstehen die in ihrer Differenzierungsfähigkeit den Monolyerzellen überlegen sind und z.B. knorpelähnliche Eigenschaften aufweisen (Anderer und Libera, 2002). Diese Eigenschaften, die möglichst genau das native Gelenkknorpelgewebe widerspiegeln, sind gekennzeichnet durch die Expression von Kollagen II (Hauptstrukturprotein der extrazellulären Matrix des hyalinen Knorpels), von Proteoglykanen, z.B. Aggrecan und dem intrazellulären chondrozytenspezifischen Protein S100. Darüber hinaus ist es wünschenswert dass die Expression von Kollagen I, welches während der Zellvermehrungsphase in der Monolayerkultur zwangsläufig hochreguliert wird, in den Zellaggregaten wieder reduziert wird da dieses Protein im nativen Gelenkknorpel praktisch nicht vorkommt. Die so generierten Zellaggregate (Sphäroide) werden seit 2002 beispielsweise von der Firma co.don angeboten um damit vor allem kleinere verletzungsbedingte Knorpeldefekte bei jüngeren Patienten zu therapieren. Dazu wird dem Patienten körpereigenes Gelenkknorpelgewebe entnommen und die daraus isolierten Zellen nach Vermehrung und 3D Kultur als Sphäroide (Chondrospheres®) transplantiert. Jedoch sind die so hergestellten Zellaggregate in ihrem Differenzierungsstatus begrenzt und zeigen häufig nur eine relative schwache lokale Expression des wichtigen Kollagens II sowie nur marginale Synthese von Proteoglykanen bei jedoch relativ starker Expression des unerwünschten Kollagen I. Um die Differenzierung dieser Zellaggregate weiter zu verbessern besteht die Möglichkeit das Kulturmedium mit bestimmten bioaktiven Substanzen anzureichern. Dazu zählen vorranging die vielfach in der Literatur beschriebenen Wachstumsfaktoren TGF-β1 - 3 sowie L-Ascorbinsäure (Vitamin C) als Kofaktor für die Kollagensynthese. Damit kann erreicht werden in den Zellaggregaten die in Gegenwart dieser Faktoren kultiviert werden, vor allem die Proetoglykan- und Kollagen II Synthese zu verstärken. Allerdings reicht diese biochemische Stimulation zum einen oftmals nicht aus um die Differenzierung der Knorpelzellen im 3D Zellaggregat soweit zu induzieren dass knorpeltypische Konstrukte entstehen und zum anderen ist der Einsatz von Wachstumsfaktoren wie TGF-β insbesondere für eine klinische Anwendung am Menschen umstritten. Proteine der TGF-β- Familie regulieren eine Vielzahl zellulärer Prozesse wie z.B. Proliferation, Differenzierung, Wachstum, Migration, etc., können aber auch pathologisch an der Tumorentstehung beteiligt sein bzw. das Wachstum bereits bestehender Tumoren fördern sowie deren Metastasierung begünstigen.

Nicht bekannt im Stand der Technik ist der Einsatz der Fusionskultivierungstechnik zur gezielten, verstärkten Stimulation der Differenzierung von menschlichen Zellen verschiedensten Ursprungs, vor allem Zellen sämtlicher muskoskelettaler Gewebe (nicht nur Knorpelzellen). In DE 100 13 223 und Anderer und Libera, 2002 wurde zwar bereits die Vereinigung von zwei einzelnen Sphäroiden zu einem größeren Aggregat offenbart, jedoch nicht die Aggregation von vorzugsweise 5 oder mehr Sphäroiden in einer 3D-Umgebung mit dem Hintergrund, dadurch die Redifferenzierung der Zellen zu erreichen, die mit einer gesteigerten Produktion knorpelspezifischer extrazellulärer Matrixbestandteile wie Kollagen II und Proteoglykanen einhergeht. Die im Stand der Technik offenbarte Aggregation diente vielmehr dazu, die Generierung eines größeren Zellaggregates zu ermöglichen.

Die vorliegende Erfindung zur Herstellung von in vitro Geweben durch Koaleszenz (Verschmelzung) mehrerer (5) kleinerer Zellaggregate stellt gezielt größere klinisch anwendbare Gewebe zur Verfügung, deren Struktur und charakteristische Proteinausstattung dem menschlichen Originalgewebe sehr ähnlich ist. Die spezielle Anordnung der Zellen an der Oberfläche von Einzelsphäroiden und die resultierenden Zell-Zellkontakte bei Berührung mit benachbarten Sphäroiden werden dabei als neue Differenzierungsinduktur (Verstärker) genutzt. Dabei besteht ein wesentlicher Anspruch dieser neuen Technik zur in vitro Gewebekultivierung in der Bereitstellung von größeren transplantierbaren Geweben die sich am Vorbild des jeweiligen natürlichen Körpergewebes (im konkreten Fall am Gelenkknorpel) orientieren. Diese Technik ist aber im Prinzip auch auf alle anderen Gewebe übertragbar und damit universell einsetzbar.

Die vorliegende Erfindung ermöglicht es, Knorpeldefekte verschiedener Größe z.B. im Kniegelenk mit einer geringeren Anzahl größerer, besser differenzierter und funktionaler in vitro Gewebe auszukleiden und so zu regenerieren. Ein weiterer wichtiger Aspekt des erfindungsgemäßen funktionalen Fusionsgewebes ist, dass mit dem erfindungsgemäßen Verfahren der Fusionskultur eine Möglichkeit gefunden werden konnte, bereits ohne den Einsatz differenzierungsfördernder Faktoren wie TGF-β, beachtliche Knorpeleigenschaften des nativen Gewebes auch in den funktionalen in vitro Geweben zu erzeugen. Um dies zu demonstrieren wurde die Fusionskultur mit biochemischen Stimuli kombiniert um einen synergistischen Effekt hinsichtlich der knorpelspezifischen Differenzierung zu zeigen. Viel wichtiger dabei ist jedoch, dass die Fusionskultur allein im Basalmedium (ohne jeglichen Zusatz von Wachstumsfaktoren, Ascorbinsäure oder den Einsatz synthetischer Gerüstmatrices) die Differenzierung der Knorpelzellen im Gewebe effektiv induzieren konnte und somit den einzelnen Zellaggregaten (einzelne Sphäroide) deutlich überlegen war. Damit ist die Herstellung einer neuen Generation transplantierbarer Gewebe für eine therapeutische Anwendung mit und ohne zusätzliche Wachstumsfaktoren möglich.

Eine andere Anwendungsmöglichkeit betrifft die Verwendung der in vitro hergestellten funktionalen Fusionsgewebe als Basis für eine Plattformtechnologie zum Testen von Substanzen beispielsweise in der pharmazeutischen Industrie, der chemischen Industrie, der Lebensmittelindustrie. Beispielsweise könnte damit im Bereich der rheumatischen Erkrankungen ein Testsystem bereitgestellt werden um die Physiologie von Knorpelzellen in gesunden und in arthrotischinduzierten Gewebeimitaten zu analysieren und zu vergleichen, insbesondere auch im Hinblick auf das Ansprechen auf vermeintlich therapeutische Substanzen.

Die Generierung körperähnlicher Gewebe durch die Fusion einzelner Zellaggregate ist in vitro mit dem Verfahren möglich. Ferner konnte gezeigt werden, dass die isolierten Chondrozyten aus verschiedenen Patienten nach der Dedifferenzierungsphase im Monolayer allein durch die Fusionskultur ohne den Zusatz bioaktiver Stimulanzien wieder redifferenziert werden konnten. Dadurch eröffnet sich auch ein Lösungsansatz für das vielfach auftretende Problem der Donorabhängigkeit so dass Gewebe mit replizierbaren Eigenschaften und ähnlicher Qualität mit Zellen verschiedener Spender generiert werden können.

Diesbezüglich sind die gewonnen Erkenntnisse nicht nur für die Regeneration von Gelenkknorpelgewebe anwendbar, sondern auch auf sämtliche Gewebe muskoskelettalen Ursprungs übertragbar.

Außerdem ermöglicht die Verschmelzung mehrerer Zellaggregate (Sphäroide) zu verschieden großen Geweben eine deutlich vielfältigere Formmodellierung so dass die gebildeten in vitro Gewebe (funktionale Fusionsgewebe) beispielsweise ideal auf die entsprechende Größe und Form des jeweiligen Defekts angepasst und auch spezielle Formen wie z.B. Meniskusgewebe konstruiert werden könnten. Um die Gewebefunktionalität und die Qualitätsmerkmale des funktionalen Fusionsgewebes weiter zu verbessern, können andere bekannte Methoden zur Stimulation der Differenzierung ggf. zusätzlich eingesetzt werden. Dazu zählt, insbesondere für Knorpelgewebe, die mechanische Stimulation z.B. über Druckapplikation (Kompression und Dekompression) oder die Kultivierung in speziellen Bioreaktoren und Spinner Flaschen um so die natürlichen Vorgänge und wirkenden Kräfte im Körper beispielsweise beim Laufen zu simulieren. Dies sollte einerseits das in vitro Gewebe optimal mit Nährstoffen versorgen und andererseits auch eine Weiterleitung externer Signale (Mechanotransduktion) gewährleisten was wiederum bekanntermaßen die Synthese extrazellulärer Matrixkomponenten anregen sollte.

Die Erfindung betrifft ein Verfahren zur Herstellung von in vitro Geweben durch Fusion einzelner vorgeformter Zellaggregate mit Potential zur klinischen Anwendung als autologes Transplantat für die Gewebeersatztherapie sowie als Basis für ein pharmakologisches Testsystem. Die Bereitstellung von geeigneten in vitro Geweben für die Grundlagenforschung, die klinische Forschung und letztendlich für den Menschen ist eine wissenschaftliche und technische Herausforderung. Besonders in einer Gesellschaft in der die Lebenserwartung der Menschen stetig steigt, sieht sich das Gesundheitswesen zunehmend mit degenerativen Erkrankungen der Gelenke und des gesamten Bewegungsapparats, z.B. durch arthrotische Abbauprozesse, konfrontiert. Somit besteht in Klinik und Forschung ein großer Bedarf an im Labor hergestellten funktionalen Ersatzgeweben. Im bisherigen Stand der Technik haben sich auf diesem Gebiet die erwähnten Zellaggregate in Form von Sphäroiden etabliert, die jedoch in ihrer Regenerationskapazität und der behandelbaren Defektgröße klar eingeschränkt sind. Die nun mit der Fusionskultivierung herstellbaren funktionalen Fusionsgewebe stellen unter den Techniken des "Tissue Engineering" ein "Upgrade", eine Verbesserung der einzelnen Zellaggregate (Sphäroide, Fusionen mit nur 2 Sphäroiden) dar. Mit dem erfindungsgemäßen Verfahren ist es möglich differenzierte, formbare Körpergewebe verschiedener Größe zu generieren. Die erfinderische Tätigkeit besteht grundsätzlich in der Entwicklung von anwendbaren Geweben zur Nachbildung von Körpermaterialen verschiedenen muskoskelettalen Ursprungs z.B. für den Einsatz in der regenerativen Medizin und in der pharmazeutischen Industrie, zur Untersuchung und zum besseren Verständnis von Erkrankungsmechanismen und als Plattform zum Testen neuer Medikamente.

Im Rahmen dieser Erfindung wurde ein Modell für die In-vitro-Chondrogenese mit Implikationen für sowohl die Grundlagenforschung als auch klinische Ansätze erstellt. Es wurden humane Chondrozyten verwendet, um knorpelartige dreidimensionale In-vitro-Mikrogewebe unter Verwendung des speziellen Kultursystems zusammen mit die Differenzierung fördernden bioaktiven Molekülen, jedoch ohne Zuhilfenahme jedweden Gerüstmaterials zu erzeugen. Nach der Expansion isolierter Chondrozyten in einer Monolayerkultur, begleitet von der Dedifferenzierung der Zellen, waren adäquate chondrogene Stimuli zur Redifferenzierung erforderlich. Einerseits durch Kultivieren von Chondrozyten in einer 3D-Umgebung und andererseits durch Zugabe von Wachstumsfaktoren, wie transformierendem Wachstumsfaktor-beta-2 (TGF-β2) und L-Ascorbinsäure, als Antioxidans für die Prolylhydroxylase, die für eine einwandfreie Kollagensynthese wichtig ist.

Da die mesenchymale Kondensation einer der frühesten Schritte während der Entwicklung vieler Gewebe, wie Knochen, Muskeln, Nieren oder Knorpel, ist, ahmt die Kultivierung von humanen Chondrozyten in einer 3D-Umgebung in vitro in Form von Sphäroiden diesen Aggregationsprozess von mesenchymalen Zellen als den ersten Schritt der embryonalen Chondrogenese nach (Hall und Miyake, 2000). Darüber hinaus stellt die Kombination von mehreren einzelnen Sphäroiden mit fusionierten Geweben als ein zweiter Aggregationsschritt eine neue Methode bereit, die offensichtlich die Differenzierung von knorpelartigen In-vitro-Geweben noch weiter fördert. Um die Qualität der erzeugten knorpeligen Konstrukte zu evaluieren, wurden Differenzierungsmarker verwendet. Von diesen Markern ist Kollagen Typ II das kennzeichnende Protein für Hyalinknorpel, wohingegen die Expression von Kollagen Typ I dazu verwendet wird, dedifferenzierte Regionen, die fibrösem Bindegewebe ähneln, in den In-vitro-Geweben nachzuweisen. Darüber hinaus ist ein hoher Gehalt an Proteoglykanen für funktionalen Knorpel wichtig und auch das kleine intrazelluläre Protein S100 ist ein nützlicher Indikator für differenzierte Knorpelzellen. Obwohl S100 eher selten als Marker für Chondrozyten verwendet wird, kann es dabei helfen, Knorpel- und Chondrozytendifferenzierung zu demonstrieren, da bekannt ist, dass eine verminderte S100-Expression in humanen Gelenkchondrozyten mit kumulativen Populationsverdopplungen korreliert. Die verschiedenen S100-Proteine, z. B. S100A1 und S100B, sind an einem breiten Spektrum von intrazellulären Prozessen, wie Zell-Zell-Kommunikation, Zellform, Zellstruktur und -wachstum, jedoch auch an der intrazellulären kalziumabhängigen Signaltransduktion beteiligt, und es ist von ihnen bekannt, dass sie sich Zytokinen ähnlich verhalten.

Die Chondrozyten wurden für etwa 40 Tage in einer 3D-Umgebung kultiviert, die partiell mit Differenzierungsfaktoren angereichert war. Die konstruierten knorpeligen Mikrogewebe wurden histologisch und immunhistochemisch analysiert, um die Menge und Verteilung von gewebespezifischen Matrixkomponenten zu bestimmen. Da das Vorliegen von Knorpelmatrixmolekülen und wichtigen Markern auf der Proteinebene für die Qualitätsbeurteilung der erzeugten Mikrogewebe von größerer Bedeutung ist, waren histologische und immunhistochemische Nachweistechniken in situ die Methoden der Wahl. Es wurde die In-vitro-Chondrogenese durch adulte humane Chondrozyten in einem neuen dreidimensionalen "Zwei-Schritt"-Gewebeerzeugungssystem sowie die Bildung von knorpelartigen Mikrogeweben ohne Verwendung jedweden Gerüsts oder tragenden Gelmaterials demonstriert. Die erfindungsgemäßen Fusionsgewebe können ein nützliches Modellsystem für das Studium sowohl des Metabolismus der betreffenden Zellen, z.B. von Chondrozyten und deren Aktivität in einer dreidimensionalen Konfiguration sein. Darüber hinaus sind die erzeugten Fusionsgewebe, z.B. Knorpelmikrogewebe bei einer autologen Anwendung als Transplantate geeignet, insbesondere für traumatische Defekte.

Mit dem erfindungsgemäßen Verfahren (auch "Tissue-Engineering-System") bieten sich Möglichkeiten zur Erzeugung funktionaler humaner Fusionsgewebe, insbesondere Knorpelmikrogewebe, die auf In-vitro-Studien z.B. der Chondrogenese und die therapeutische Knorpelregeneration in vivo anwendbar sind. Die Verwendung von autologen Zellen zur Konstruktion von Knorpel weist den Vorteil auf, dass das Risiko einer immunologischen Abstoßung und von übertragbaren Krankheiten verringert wird.

Im Zellzyklus arretierte Chondrozyten, die aus Knorpelgewebe isoliert worden waren, begannen erneut, sich in einer Monolayerkultur zu vermehren. Wenn sie als Monolayer serienkultiviert wurden, hörten sie auf, knorpelspezifische Makromoleküle zu synthetisieren, und ersetzten sie durch Moleküle, die normalerweise von mesenchymalen Zellen von anderen Bindegeweben exprimiert werden.

Es wurde von vielen Versuchen berichtet, einen chondrozytenspezifischen Phänotyp in verschiedenen Kultursystemen zu konservieren. Gute Ergebnisse wurden erzielt, indem humane Chondrozyten auf biologisch abbaubaren Gerüsten (Scaffolds) gezüchtet wurden. Die meisten synthetischen Polymermatrizes neigen jedoch dazu, sich bei einem deutlich sauren pH-Wert zu zersetzen, was sich als schädlich für implantierte Zellen und umliegende Gewebe erwiesen hat. Des Weiteren ist der Einfluss des Gerüstmaterials auf das Chondrozytenverhalten und die Zell-Zell-Wechselwirkungen oftmals schwer zu erfassen. Im Gegensatz zu den oben erwähnten Ansätzen basiert das erfindungsgemäße Verfahren und die erhaltenen Fusionsgewebe auf einer gerüstfreien Induktion von knorpelartigen In-vitro-Geweben, wobei die Nachahmung des ersten Schritts der In-vivo-Chondrogenese ausgenutzt wird. Die Zellen werden nicht in eine feststehende Gerüststruktur gezwängt, was eine bessere Einpassung in einen gegebenen Gelenkknorpeldefekt ermöglichen kann. Viele der zitierten Berichte demonstrieren im Allgemeinen eine viel versprechende Redifferenzierung von tierischen Chondrozyten durch 3D-Kultursysteme. Die Erzeugung von dreidimensionalen Knorpelgewebestrukturen aus adulten humanen Chondrozyten ist jedoch eine viel größere Herausforderung. In enormen Zahlen aus gesunden Gelenken von Tieren isolierte Chondrozyten können direkt für 3D-Kulturen verwendet werden, wohingegen In-vitro-Studien mit humanen Chondrozyten in den meisten Fällen Monolayer-Expansionsschritte erfordern. Chondrozyten von verschiedenen Kniegelenken von Tieren unterscheiden sich bereits in Bezug auf ihre In-vitro-Biologie von Zellen, die aus der entsprechenden Oberfläche von Gelenken vom Menschen isoliert wurden.

Die resultierenden einzelnen Sphäroide oder Fusionen bei Verwendung von humanen Chondrozyten stellen eine allmähliche Redifferenzierung von Zellen in diesen Mikrogeweben dar. Dieser Redifferenzierungsprozess wurde biochemisch beeinflusst, indem das Medium mit TGF-β2 supplementiert wurde. In Bezug auf Knorpel wird TGF-β funktional freigesetzt und die entsprechenden Rezeptoren werden ebenfalls in Chondrozyten exprimiert. Eine Reihe von Studien hob die Rolle von TGF-β und insulin-ähnlichem Wachstumsfaktor-1 (IGF-I) als wichtige Mediatoren bei der Förderung der Gewebereparatur durch erhöhte Produktion von Hauptkomponenten der Gelenkknorpelmatrix hervor. Die Auswirkung von TGF-β auf den Matrixmetabolismus in Chondrozyten ist jedoch umstritten. Widersprüchliche Berichte haben Anstiege und Abnahmen der Proteoglykansynthese, eine Verstärkung der Differenzierung und eine starke Zunahme oder eine Hemmung des Wachstums demonstriert. In unserer Studie stellte sich TGF-β2 als ein wirksamer Promotor der chondrogenen Differenzierung in den 3D-Kultursystemen heraus. Eine Kombination von TGF-β2 mit L-Ascorbinsäure in dem Kulturmedium führte in Bezug auf die chondrogene Redifferenzierung in Mikrogeweben zu ähnlichen Ergebnissen. Von der Supplementierung mit Ascorbat wird häufig berichtet, dass sie die Differenzierung begünstigende Effekte im Hinblick auf die chondrogene Prägung vermittelt. Die Auswirkung von Ascorbinsäure auf die Proteoglykansynthese durch Chondrozyten wird kontrovers diskutiert. Die Ergebnisse zeigen, dass Ascorbinsäure allein die Proteoglykansynthese nicht stimulieren konnte. Safranin-O-Positivität wurde in Fusionen nachgewiesen, die mit oder ohne Ascorbinsäure kultiviert wurden, was darauf hindeutet, dass der Stimulus von der Fusionskultur anstelle von dem Ascorbat selbst induziert wurde. Es wird allgemein angenommen, dass Ascorbat die Kollagenproduktion durch seine Auswirkung auf die Prolylhydroxylierung moduliert. Interessanterweise wurde Kollagen Typ II durch Zugabe von L-Ascorbinsäure allein weder in einzelnen Sphäroiden noch in Fusionen vermehrt. Im Gegensatz dazu wurde Kollagen Typ I in einzelnen Sphäroiden, die in ascorbathaltigem Medium kultiviert wurden, im Vergleich zu den in Basalmedium kultivierten stärker exprimiert. Dieses Phänomen deutet darauf hin, dass L-Ascorbinsäure die Kollagensynthese oder -assemblierung im Allgemeinen anstelle von Kollagen Typ II im Besonderen stimulierte und Ascorbat die Produktion von dem Kollagentyp fördert, auf dessen Expression die Transkriptionsmaschinerie der Zellen programmiert ist. Da in einer Monolayerkultur expandierte, dedifferenzierte Chondrozyten zum Starten der 3D-Kultur verwendet wurden, kann es möglich sein, dass die Zellen zur Synthese von Kollagen Typ I neigen, die wiederum von der Gegenwart von L-Ascorbinsäure begünstigt wurde. Die Kombination mit TGF-β2 schien diesen Prozess umzukehren, da TGF-β2 die Genexpression von Kollagen Typ II selektiv auslösen kann. Die Proteinexpression in einzelnen Sphäroiden und fusionierten Mikrogeweben veränderte sich in Richtung einer verbesserten chondrogenen Differenzierung, was durch eine erhöhte Expression von Kollagen Typ II und S100 dargestellt wurde, die von einer Herabregulation von Kollagen Typ I begleitet wurde, das auf die Ränder der Mikrogewebe beschränkt war. Ein zusätzlicher die Differenzierung verstärkender Effekt allein durch unsere neue Fusionskulturtechnik war im Fall der Expression von Kollagen Typ II und S100 ebenfalls ersichtlich. Dieses Zwei-Schritt-Aggregationssystem, das durch Fusionieren von vorgeformten Aggregaten implementiert wurde, war ein neuer Stimulus, der in der aktuellen Studie angewendet wurde, um knorpelartige Mikrogewebe zu erzeugen, und der vermutlich auf Zell-Zell- und/oder Zell-Matrix-Wechselwirkungen beruht. Die Fusionskultivierung verbesserte in jeder Medienbedingung die Knorpelgewebebildung im Vergleich zu einzelnen Sphäroiden. Der die Differenzierung fördernde Effekt in Fusionen ist die Zellkondensation, sie ist das Schlüsselstadium bei der Entwicklung von Skelettgeweben, die die selektive Regulation von für die Chondrogenese spezifischen Genen erleichtert. Fibronektin und TGF-β sind an der Kondensationsbildung beteiligt, insbesondere während der Inititierungsphase. TGF-β reguliert eine Reihe von Molekülen hinauf, die mit prächondrogenen Kondensationen assoziiert sind, einschließlich Tenascin, Fibronektin, N-CAM und N-Cadherin. Zell-Zell-Wechselwirkungen und von Gap Junctions abhängige Kommunikation sind ausschlaggebend an der chrondrogenen Differenzierung beteiligt. Adulte Gelenkknorpelchondrozyten existieren als einzelne Zellen, die in der extrazellulären Matrix eingebettet sind, und direkte intrazelluläre Kommunikation über Gap Junctions findet vorwiegend unter den abgeflachten Chondrozyten statt, die der äußeren Knorpelschicht zugewandt sind. Chondrozyten, die aus adultem Gelenkknorpel extrahiert und in einer Primärkultur gezüchtet wurden, exprimieren jedoch Connexin 43 (Cx43) und bilden funktionale Gap Junctions, die die Ausbreitung von interzellulären Kalziumwellen aufrechterhalten können. Diese Mechanismen und Komponenten, die für die Zellkondensation und -kommunikation relevant sind, können dabei helfen, den überlegenen chondroinduktiven Effekt unserer eingeführten Fusionskultur zu erklären. Beim Starten des Fusionsprozesses wurden mehrere Schichten der Oberflächen von In-vitro-Geweben in engem Kontakt zueinander angeordnet, wodurch Zell-Zell-Wechselwirkungen und die von Gap Junctions vermittelte Kommunikation ermöglicht und dadurch damit verbundene Prozesse beeinflusst wurden, wie veränderter Austausch von Kalziumionen oder sekundären Messengern, was zur Förderung der Knorpeldifferenzierung führte. Im Hinblick auf die mesenchymale Kondensation als einen der frühesten Schritte während der Knorpelentwicklung in vivo ahmen die einzelnen Sphäroide diesen Prozess in vitro gut nach. Die Kombination von mehreren einzelnen Sphäroiden mit fusionierten Mikrogeweben als ein zweiter Aggregationsschritt fördert die Nachahmung dieses Entwicklungsstadiums der embryonalen Knorpelbildung weiter.

S100 ist ein Marker in humanen Chondrozyten. Die Überführung von frisch isolierten postmitotischen Chondrozyten in eine Monolayerkultur führte zu einem Neustart der Proliferation, begleitet von einer Verringerung und dem Stopp der Kollagen-Typ-II-Expression in den ersten Passagen, wohingegen S100-Protein nach 4 Populationsverdopplungen(PD) oder sogar bis zu mehr als 22 PD nachgewiesen werden konnte. Parallel dazu wurde Kollagen Typ I progressiv exprimiert, was bereits in Zellen der Passage 2 begann. Die Selbstaggregation von dedifferenzierten, proliferierenden Zellen führte zu einem Proliferationsstopp und der anschließenden Expression des S100-Proteins, unabhängig von der Medienzusammensetzung und der Kulturbedingung. Die Expression von S100 war in Gegenwart von TGF-β und in allen mit der Fusionstechnik erzeugten Mikrogeweben sogar noch höher. Im Gegensatz dazu waren Antikörper gegen Kollagen Typ II kaum dazu in der Lage, dieses Protein in einzelnen Sphäroiden in Basalmedium und in mit Ascorbat angereichertem Medium nachzuweisen. Um Kollagen Typ II in Mikrogeweben zu exprimieren, war eine erweiterte Stimulation des Differenzierungsprozesses erforderlich, d. h. durch Fusionsbildung und/oder biochemisch. Das beschriebene sequentielle Auftreten der Chondrozyten-Markerproteine S100 und Kollagen Typ II in differenzierenden Mikrogeweben passt auch in den Kontext, dass S100-Proteine Ziele des Trios der SOX-Transkriptionsfaktoren (SOX9 und dessen Koaktivatoren SOX5 und SOX6) sind und dass der Transkriptionsfaktor SOX9 wichtige Rollen in aufeinander folgenden Schritten des Chondrozyten-Differenzierungswegs spielt. Die vorgelegten Daten und die zitierte Literatur offenbaren, dass es offensichtlich üblich ist, das S100-Protein als einen frühen chondrospezifischen Zellmarker zu verwenden, der zur Qualitätskontrolle von Zellen in Kultur und sogar in In-vitro-Geweben nützlich wäre. Darüber hinaus ist S100 zur Unterscheidung von Chondrozyten von anderen mesenchymalen Zellen in Bindegeweben, wie Osteoblasten oder Fibroblasten, ideal geeignet, was ein Ausschließen einer Verunreinigung der Zellen in hergestellten knorpelartigen Gewebekonstrukten ermöglicht. Die Koexpression von Kollagen Typ II und S100 auf der Proteinebene in bestimmten Regionen von Mikrogeweben wurde in einzelnen Sphäroiden und Fusionen, die in TGF-β2-haltigem Medium kultiviert worden waren, deutlich sichtbar, was Immunfluoreszenz als Methode der Wahl rechtfertigt. Ein weiteres Beispiel, das die Vorteile des Nachweisens von Proteinen mittels Immunfluoreszenz demonstrierte, war der markante, für Kollagen Typ I positive Außenrand in Kryoschnitten von einzelnen Sphäroiden und Fusionen in Gegenwart von TGF-β2. In diesen Regionen fehlte Kollagen Typ II praktisch, was eine kleine Oberflächenschicht, die fibrösem Bindegewebe ähnelt, darstellt, was Ähnlichkeiten zur Zusammensetzung von nativem Gelenkknorpel widerspiegelt, die wiederum nur mithilfe einer immunhistochemischen Technik nachweisbar war.

Die Kultivierung von expandierten und dedifferenzierten humanen Gelenkchondrozyten in einer dreidimensionalen Umgebung führt zur Bildung von Mikrogeweben in Form von einzelnen Sphäroiden oder Fusionen und hat die Redifferenzierung von Zellen in jenen knorpelartigen In-vitro-Gewebekonstrukten zur Folge. Die Relevanz von S100 als einen Marker für die frühe Chondrozytendifferenzierung wurde durch eine zum ersten Mal beschriebene "Expressionsverschiebung" im Vergleich zu Kollagen Typ II offenbart, d. h. späte Herabregulation der S100-Expression während der Dedifferenzierung in einer Monolayerkultur, aber frühe und einfachere (nur durch Aggregation in Abwesenheit von Stimulationsfaktoren) Hinaufregulation während der Redifferenzierung in einer 3D-Kultur, bevor Kollagen Typ II überhaupt reexprimiert wurde.

Die vorliegende Erfindung stellt Daten bereit, dass das erfindungsgemäße Verfahren ("Fusionskulturtechnik") die chondrogene Differenzierung in vitro fördert, wodurch die Einrichtung einer durch die Zellen selbst hergestellten extrazellulären Matrix eingeleitet wird, die sich vorwiegend aus Kollagen Typ II und Proteoglykanen zusammensetzt. In einzelnen Sphäroiden lag nur eine geringfügige Expression dieser Knorpelmarker in Basalmedium und in Gegenwart von L-Ascorbinsäure vor. Diese Einschränkung der Matrixproduktion konnte durch die Bildung von fusionierten Mikrogeweben überwunden werden. Das erfindungsgemäße Verfahren zusammen mit geeigneten stimmulatorischen Wachstumsfaktoren induzierte synergistisch die Reexpression des Knorpelphänotyps und stellt eine Plattformtechnologie zur Erzeugung von gerüstfreien Transplantaten für Menschen in vitro bereit, die klinisch auf die Regeneration von traumatischen Knorpeldefekten oder auf Osteoarthritis anwendbar sind. Das funktionale Fusionsgewebe ("fusionierte Mikrogewebe") stellt gelenkknorpelartige Transplantate dar, die therapeutische Implikationen bei der Bekämpfung von einigen der wichtigsten rheumatischen Erkrankungen haben können.

Die nachfolgende Figuren und Beispiele dienen der Erläuterung der Erfindung ohne die Erfindung jedoch auf die Figuren und Beispiele einzuschränken.

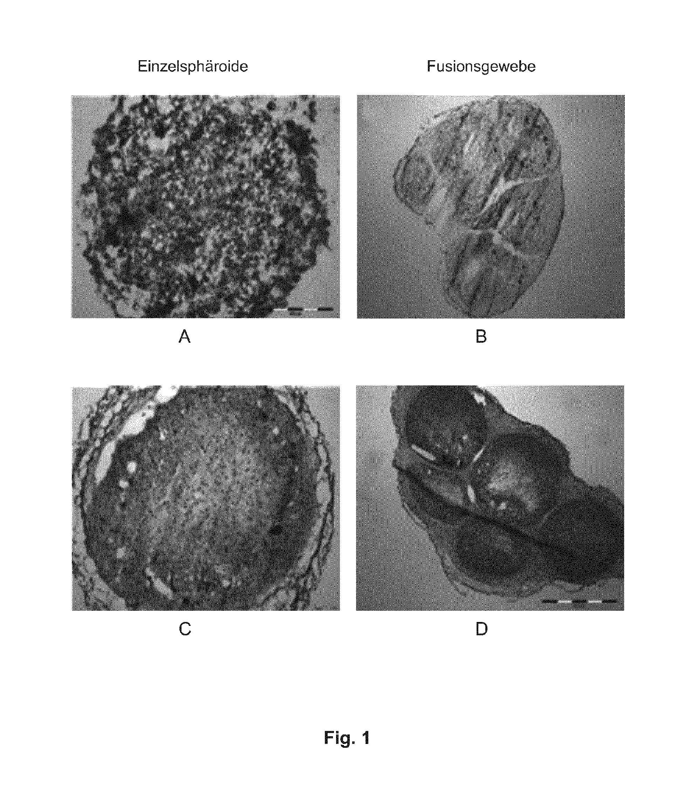

Figur 1: Darstellung der Qualitätssteigerung durch Herstellung von Fusionsgewebe aus humanen Chondrozyten anhand der Bildung einer knorpelspezifischen extrazellulären Matrix. Der Nachweis von für Knorpelgewebe charakteristische Proteoglykane in Einzelsphäroiden und Fusionsgeweben erfolgte mittels Safranin O-Färbung (rot = Proteoglykane).
A: Einzelsphäroid im Basalmedium zeigt keine Proteoglykansynthese (keine Rotfärbung). B: Fusionskultur von 5 Einzelsphäroiden in Basalmedium induziert die Produktion von Proteoglykanen und Einbau in die extrazelluläre Matrix des Fusionsgewebes (Rotfärbung). C: Einzelsphäroid in Basalmedium angereichert mit TGF-β2 fördert Proteoglykanbildung. D: Fusionsgewebe in Gegenwart von TGF-β2 stimuliert die Synthese und Sekretion von Proteoglykanen noch deutlich stärker (intensive Rotfärbung).

Einzelsphäroide (A und C) wurden mit einer höheren Vergrößerung aufgenommen; Durchmesser ca. 1000 - 1300 µm.

Fusionsgewebe wurden mit einer geringeren Vergrößerung aufgenommen;

Maße: ca. 2000 x 3000 µm.

### Beispiele

### Beispiel 1: Zellquelle und Monolayerkultur von humanen Gelenkchondrozyten

Gelenkknorpel wurde von humanen Femurkondylen von Patienten bezogen, die einer Knieoperation unterzogen worden waren. Die Ergebnisse wurden durch Durchführung von drei unabhängigen Versuchen mit Knorpel von drei unterschiedlichen Patienten erreicht. Knorpelgewebe wurde mit einem scharfen Skalpell von den Kondylen abgeschält und Chondrozyten wurden durch mechanische Zerkleinerung des Gewebes mit einem Skalpell, gefolgt von enzymatischer Behandlung von der umgebenden Matrix isoliert. Dazu wurde das gewürfelte Gewebe in Alpha-Medium und HAMs F12 (1:1) mit Kollagenase Typ II (350 E/ml) gegeben. Das geschlossene Röhrchen wurde in einen Schüttler unter Intervall-Mischen mit 300 U/min gestellt und wurde für 20 h bei 37 °C inkubiert. Die extrahierten Chondrozyten wurden bei 300 x g für 5 min zentrifugiert. Der Überstand wurde entfernt und das Pellet wurde mit 10 ml Alpha-Medium plus HAMs F12 resuspendiert, das mit 1 % L-Glutamin und 10 % Humanserum (Serumpool von freiwilligen Spendern) angereichert war und im weiteren als Basalmedium bezeichnet wird. Die Chondrozyten wurden in einer Zelldichte von 2 x 10⁴ Zellen/cm² ausplattiert. Die Zellen wurden in einer Monolayerkultur bei 37 °C und 5 % CO₂ für zwei Passagen expandiert.

### Beispiel 2: Erzeugung von knorpelartigen Mikrogeweben

Um die Mikrogewebebildung zu induzieren, wurden Chondrozyten in mit Agarose beschichteten Wells von 96-Well-Platten in einer Konzentration von 3 x 10⁵ Zellen/Well in 200 µl Basalmedium pro Well gesät. Nach zwei Tagen hatten sich bereits stabile Chondrozytenaggregate gebildet, die dann unter anderen Bedingungen kultiviert wurden, um die Redifferenzierung zu fördern.

### Beispiel 3: Erzeugung von Fusionsgeweben und Bedingungen für die chondrogene Redifferenzierung

Neben den speziellen Kultivierungsbedingungen, die das Fusionieren von einzelnen Sphäroiden miteinander ermöglichen, wurden außerdem bestimmte bioaktive Substanzen verwendet, um die Redifferenzierung zu verstärken. Die Induktion von fusionierten Aggregaten wurde durch Kombinieren von fünf einzelnen Sphäroiden in einem Well einer 96-Well-Platte erzielt. Die einzelnen Sphäroide und die Fusionsgewebe wurden unter vier unterschiedlichen Bedingungen hinsichtlich des Vorliegens von bioaktiven Molekülen kultiviert. Die In-vitro-Aggregate wurden entweder in Basalmedium, das im Weiteren als BM abgekürzt wird, in mit 50 µg/ml L-Ascorbinsäure supplementiertem BM, in BM plus 5 ng/ml TGF-β2 oder in mit 50 µg/ml L-Ascorbinsäure und 5 ng/ml TGF-β2 supplementiertem BM kultiviert. Die Gesamtkultivierungszeit für alle Mikrogewebe in den erwähnten Bedingungen war sechs Wochen, wobei das Medium im Fall der einzelnen Sphäroide dreimal pro Woche und im Fall der fusionierten Mikrogewebe jeden Tag ausgetauscht wurde.

### Beispiel 4: Analyse von In-vitro-Knorpel

Nach 6 Wochen wurden die In-vitro-Gewebekonstrukte geerntet und für die weitere Analyse präpariert. Der Konstruktdurchmesser wurde mittels Bildanalyse berechnet. Die Morphologie wurde mit einem inversen Mikroskop mit Phasenkontrast CKX 41, einer Digitalkamera DP 71 und der Bildanalysesoftware Cell^{F} beurteilt. Die Gewebekonstrukte wurden in PBS gespült, in Neg-50-Gefrierschnitt-Medium eingebettet und unter Verwendung eines Kryomikrotoms geschnitten. Die 7-µm-Kryoschnitte auf Glasobjektträgern wurden an der Luft getrocknet und direkt analysiert oder bei - 20°C gelagert.

### Beispiel 5: Histologie und Immunhistochemie

Vor den Analysen wurden die Monolayer-Chondrozyten von Passage 2 und die Gewebekryoschnitte auf den Glasobjektträgern in einem Zwei-Schritt-Verfahren fixiert. Zunächst wurden sie mit 4%-igem Formaldehyd bei 4 °C für 10 min fixiert. Anschließend wurden die Objektträger in einem 1:1-Gemisch von Methanol und Aceton bei -20 °C für 10 min inkubiert. Nach diesem Fixierungsvorgang wurden die Objektträger für 3 bis 5 min in PBS gespült. Eine histologische Färbung mit Hämatoxylin und Eosin wurde zur morphologischen Analyse der Zellen und Gewebe und mit Safranin O-Fast Green zum Nachweis von Glykosaminoglykanen (GAG) durchgeführt. Die fixierten Chondrozyten und Kryoschnitte wurden immunhistochemisch für humanes Kollagen Typ I, Typ II und S100 gefärbt. Die Objektträger wurden mit PBS gespült und für 20 min bei Raumtemperatur (RT) mit Ziegennormalserum, das 1:50 in PBS/0,1%-igem RSA verdünnt worden war, inkubiert, um unspezifische Bindungen zu blockieren. Primäre Antikörper wurden wie folgt in PBS/0,1%-igem RSA verdünnt: Anti-Kollagen-Typ-I (1:1000), Anti-Kollagen-Typ-II (1:1000) und Anti-S100 (1:400). Die Zellen und die Kryoschnitte wurden mit den primären Antikörpern über Nacht bei 4 °C in einer Feuchtkammer inkubiert. Die Objektträger wurden dreimal mit PBS gewaschen und dann für 1 h im Dunkeln bei RT in einer Feuchtkammer mit Cy3-konjugiertem Ziege-Anti-Maus-Antikörper (Kollagen Typ I und II) und Ziege-Anti-Kaninchen-Antikörper (S100), die 1:600 in PBS/0,1%igem RSA mit DAPI (1 µg/ml) verdünnt worden waren, inkubiert, um die Zellkerne zu färben. Die Objektträger wurden dreimal mit PBS gewaschen, und die Zellen und Gewebeschnitte wurden anschließend in Fluoreszenz- Eindeckmedium eingedeckt und mit einem Deckglas abgedeckt, um Fluoreszenzbleichung zu verhindern. Schließlich wurden die Objektträger bis zur Analyse mittels Fluoreszenzmikroskopie im Dunkeln bei 4 °C gelagert. Kryoschnitte von nativem humanem Gelenkknorpel wurden als Positivkontrolle für Kollagen Typ II und S100 und als Negativkontrolle für Kollagen Typ I verwendet. Darüber hinaus umfassten alle Versuche als Negativkontrolle den Austausch der primären Antikörper durch PBS als Kontrolle auf unspezifische Bindung des sekundären Antikörpers.

### Beispiel 6: Phasenkontrastmikroskopie für Zellkulturdokumentationen

Fotos der einzelnen Sphäroide und der Fusionen wurden in Schwarzweiß mit dem Lichtmikroskop CKX 41 aufgenommen, das mit der Kamera DP 71 ausgestattet war. Die Dokumentation erfolgte mit der Cell^{F}-Bildanalysesoftware für die Mikroskopie.

### Beispiel 7: Farbmikroskopie für histologische Proben

Die Ergebnisse der histologischen Analysen wurden unter Verwendung des Mikroskops BX 41, das mit der Kamera Color View I ausgestattet war, und der Cell^{D}-Bildanalysesoftware dokumentiert.

### Beispiel 8: Fluoreszenzmikroskopie für immunhistochemische Analysen

Die Fluoreszenz der Cy3-konjugierten Antikörper und von DAPI der immunhistochemisch gefärbten Zellen und Kryoschnitte wurde mit dem computergestützten Fluoreszenzmikroskopsystem IX81 mit einem Xenonbrenner MT20 sichtbar gemacht. Die

Bilddokumentation und -auswertung wurde mit der Digitalkamera F-View II und der Cel1^{R}-Bildanalysesoftware für die Mikroskopie durchgeführt.

### Beispiel 9: Zusammenfassung der Ergebnisse

### Beispiel 9.1: Analyse von humanem Gelenkknorpelgewebe

Proben von humanem Hyalinknorpel, die von drei verschiedenen Spendern isoliert worden waren, wurden mittels Histologie und Immunhistochemie analysiert. Die Analysen einer Probe sind repräsentativ für alle Spender im Folgenden beschrieben. Die HE-Färbung zeigt die typische Struktur von humanem Hyalinknorpel mit länglichen abgeflachten Zellen in der oberflächlichen Zone und abgerundeten Zellen, die häufig in kleinen isogenen Gruppen (Lakunen) in der mittleren Zone des Gewebes angeordnet sind. Es zeigte sich, dass die Chondrozyten (dunkelblaue Punkte stellen die Zellkerne dar) von der extrazellulären Matrix, die hellblau dargestellt ist, voneinander getrennt werden. Die SO-Fast-Green-Färbung stellt den Gehalt von Proteoglykanen im Gewebe dar. Rot gefärbte Bereiche sind Safranin-O-positiv und deuten auf Glykosaminoglykane (GAG) hin. Insgesamt war die Intensität der SO-Färbung noch immer ziemlich hoch, was auf das Vorliegen von Proteoglykanen in mindestens 70 % des Gewebes hindeutet. Oberflächenregionen des Knorpelgewebeschnitts wurden jedoch von Fast Green grün gefärbt, was impliziert, dass die Proteoglykane abgebaut wurden. Die Ergebnisse der Immunhistochemie zeigten eine überwiegende Expression von Kollagen Typ II. Die Expression dieses Hyalinknorpel-Kennzeichens war insbesondere in den Oberflächenregionen verringert, was eine Veränderung der Matrixzusammensetzung in dieser Zone bestätigt. S100-Proteine wurden von den meisten der Zellen exprimiert, was als eine positive rote Färbung zu sehen war, die auf das Zytoplasma der Zellen beschränkt war. Wie erwartet, wurde Kollagen Typ I in nativem Hyalinknorpel nicht exprimiert, mit Ausnahme einer sehr dünnen Schicht auf der Oberfläche. Vergleichbare histologische und immunhistochemische Ergebnisse wurden mit den Knorpelproben von zwei anderen Spendern erhalten (Daten nicht gezeigt).

### Beispiel 9.2: Dedifferenzierung der als Monolayer kultivierten humanen Chondrozyten

Während der Zellexpansion in der Monolayerkultur dedifferenzierten die Chondrozyten und erlangten eine Fibroblastenzellform (Daten nicht gezeigt). Immunhistochemische Analysen offenbarten, dass humane Chondrozyten in Passage 2 (p2) einer Monolayerkultur, d. h. nach etwa 4 Populationsverdopplungen (PD), Kollagen Typ II nur in seinem sehr geringen Ausmaß oder überhaupt nicht exprimierten. Im Gegensatz dazu wurde das Protein S100 noch immer in allen Zellen als typische punktierte Färbung exprimiert. Des Weiteren wurde das atypische Kollagen Typ I von dem Großteil der Zellen bereits nach einer solch kurzen Zeit in Kultur exprimiert.

### Beispiel 9.3: Erzeugung von In-vitro-Knorpelmikrogeweben durch spezielle 3D-Kultursysteme

Bedingungen einer dreidimensionalen Kultivierung führten zur Bildung von Mikrogeweben in Form von einzelnen Sphäroiden oder Fusionskulturen. Im Allgemeinen wurden stabile Sphäroide unabhängig von der Gegenwart von L-Ascorbinsäure und/oder TGF-β2 innerhalb von zwei Tagen gebildet und wurden in den folgenden 2 - 3 Wochen der Kultivierung kompakter, blieben jedoch in Bezug auf die Größe konstant, bis sie nach 6 Wochen geerntet wurden. Gewöhnlich lag der Durchmesser der einzelnen Sphäroide im Bereich von etwa 800 - 1400 µm. Abgesehen von Größenunterschieden zeigten die einzelnen Sphäroide keine erkennbaren morphologischen Veränderungen aufgrund von unterschiedlichen Mediensupplementen. Im Gegensatz dazu schien die Gegenwart von TGF-β2 und/oder L-Ascorbinsäure bei der Bildung der fusionierten Mikrogewebe einen Einfluss auf den Fusionsgrad zu haben, da in jeder Medienzusammensetzung, mit Ausnahme des Basalmediums, die einzelnen Sphäroide sich zu einem ziemlich kompakten Mikrogewebe verbanden, das ein kohärentes Aggregat darstellte. Obwohl die Sphäroide in dem Basalmedium an einigen Oberflächenregionen verschmolzen, bildeten die fusionierten Mikrogewebe ein eher lockeres Aggregat, in dem alle einzelnen Sphäroide wohl definiert und voneinander unterscheidbar blieben.

### Beispiel 9.4: Zell-/Matrixmorphologie und Proteoglykansynthese der In-vitro-Knorpelmikrogewebe

Die HE-Färbung von Kryoschnitten einzelner Sphäroide und der fusionierten Mikrogewebe (funktionales Fusionsgewebe) wurden analysiert. Im Fall des Basalmediums waren die Zell- und Matrixverteilungen in den einzelnen Sphäroiden und den Fusionen recht ähnlich, obwohl in den Fusionen Regionen mit erhöhter EZM-Produktion sichtbar waren, was durch einen größeren Abstand zwischen den Chondrozyten widergespiegelt wurde. Die Zugabe von L-Ascorbinsäure allein führte zu ungünstigen schwachen Gewebekonstrukten, was durch rissige Kryoschnitte widergespiegelt wurde. Die fusionierten Konstrukte waren jedoch wiederum kompakter, wie durch die gesteigerte EZM-Synthese zu erkennen war. Beide Medienzusammensetzungen, TGF-β2 allein oder in Kombination mit L-Ascorbinsäure, führten zu einer gesteigerten Matrixsynthese; insbesondere die Fusionen entwickelten jedoch eine Morphologie, die nativem Knorpel ähnlich war. Sehr bemerkenswert ist der äußere Ring mit hohem Matrixgehalt und ziemlich flachen Zellen in den einzelnen Sphäroiden und den Fusionen in Basalmedium, das mit TGF-β2 und L-Ascorbinsäure angereichert war. Die Gegenwart des Zytokins TGF-β2 in dem Kulturmedium induzierte unabhängig von der Fusionskulturtechnik eine gesteigerte Synthese von Proteoglykanen. Die Zugabe von TGF-β2 zusammen mit L-Ascorbinsäure führte zu den besten Ergebnissen in Bezug auf Safranin-O-Positivität. Im Gegensatz dazu schränkte die Wirkung von L-Ascorbinsäure allein die Proteoglykansynthese in den einzelnen In-vitro-Geweben eher ein, da nahezu keine GAG in diesem Medium nachweisbar waren. Andererseits ermöglichte die Kultivierung von mehreren Sphäroiden als fusionierte Mikrogewebe die Induktion der Proteoglykansynthese unabhängig von den Differenzierungsfaktoren.

### Beispiel 9.5: Immunhistochemische Analysen der In-vitro-Knorpelmikrogewebe

Das Zytokin TGF-β2 allein oder in Kombination mit L-Ascorbinsäure förderte die Redifferenzierung von Chondrozyten in 3D-Kulturen, was zu einer erhöhten Kollagen-Typ-II-Expression führte. Insbesondere induzierte die Fusionskultivierung selbst im Vergleich zu einzelnen Gewebekonstrukten eine verstärkte Synthese von Kollagen Typ II, selbst in dem Basalmedium. Die Fusionskultivierung in Kombination mit TGF-β2-Supplementierung zeigte eine sogar noch weiter erhöhte Kollagen-Typ-II-Expression, wohingegen die Supplementierung mit L-Ascorbinsäure allein im Vergleich zum Basalmedium keine sichtbare Hinaufregulation von Kollagen Typ II induzierte.

Ähnlich der Expression von Kollagen Typ II wurde auch die S100-Expression nur aufgrund der Kultivierung von einzelnen Sphäroiden als fusionierte Mikrogewebe erhöht. Wiederum trugen die Differenzierungseffekte von TGF-β2 allein oder in Kombination mit L-Ascorbinsäure zu einer Hinaufregulation der S100-Expression bei. Es ist bemerkenswert, dass die S100-Expression korrekt mit der Lokalisierung von Kollagen Typ II in den Schnitten der einzelnen Sphäroide und der Fusionen korrelierte, insbesondere in Gegenwart von TGF-β2 allein oder plus L-Ascorbinsäure. Während Kollagen Typ II im inneren Teil der Mikrogewebe stark exprimiert wurde, war seine Expression in den äußeren Zonen sehr schwach. Ähnliche Expressionsmuster wurden für S100 beobachtet, insbesondere in der Medienzusammensetzung von TGF-β2 + L-Ascorbinsäure, d. h. starke Signale in den Zentren der Gewebe, aber schwache oder sogar fehlende Signale in den äußeren Ringen.

Im Hinblick auf Kollagen Typ I als einen Marker für dedifferenzierten Knorpel zeigte sich eine verminderte Expression dieses Proteins in Gegenwart von TGF-β2 und TGF-β2 plus L-Ascorbinsäure. In sowohl den einzelnen Sphäroiden als auch den fusionierten Mikrogeweben war die Kollagen-Typ-I-Expression auf die äußere Zone der In-vitro-Gewebe beschränkt. Darüber hinaus ist es nennenswert, dass Kollagen Typ II in Regionen, in denen Kollagen Typ I hinaufreguliert wurde, nahezu fehlte und umgekehrt.

### Referenzen

Anderer, U., and Libera, J. In vitro engineering of human autogenous cartilage. J Bone Miner Res 17, 1420, 2002.
Brittberg, M., Lindahl, A., Niesson, A., Ohlsson, C., Isaksson, O., and Peterson, L. Treatment of deep cartilage defects in the knee with autologous chondrocyte transplantation. N Engl J Med 331, 889, 1994.
Hall, B.K., and Miyake, T. All for one and one for all: condensations and the initiation of skeletal development. Bioessays 22, 138, 2000.

## Patentansprüche

1. Verfahren zur Herstellung von funktionalem Fusionsgewebe
**dadurch gekennzeichnet, dass**
a) Zellen aus Gewebe humanen oder tierischen Ursprungs isoliert werden,
b) die isolierten Zellen in eine andere Umgebung gebracht und vermehrt werden,
c) aus den vermehrten Zellen Sphäroide hergestellt werden,
d) fünf oder mehr Sphäroide fusioniert werden, gegebenenfalls in Gegenwart mindestens eines weiteren Differenzierungsinduktors.

2. Verfahren zur Herstellung von funktionalem Fusionsgewebe **dadurch gekennzeichnet, dass**
a) Zellen aus solidem Gewebe humanen oder tierischen Ursprungs isoliert werden,
b) aus den isolierten Zellen dedifferenzierte Zellen hergestellt werden,
c) aus den dedifferenzierten Zellen Sphäroide hergestellt werden,
d) fünf oder mehr Sphäroide fusioniert werden, gegebenenfalls in Gegenwart mindestens eines weiteren Differenzierungsinduktors.

3. Verfahren nach einem der vorgehenden Ansprüche **dadurch gekennzeichnet, dass** der/die weiteren Differenzierungsinduktor(en) ausgewählt werden aus mechanischen, chemischen oder biochemischen Differenzierungsinduktoren.

4. Verfahren nach einem der vorgehenden Ansprüch dadurch gekennenzeichnet, dass die Kultivierung in 3D-Umgebung mit mindestens 5 Sphäroiden und in Gegenwart mindestens eines weiteren Differenzierungsinduktors durchgeführt wird, wobei der/die weitere(n) Differenzierungsinduktor(en)ausgewählt werden aus mechanischen Stimuli wie Druckapplikation, Kultivierung in Bioreaktoren wie Roting Wall Vessel oder Spinner Flaschen, Ascorbinsäure, insbesondere L-Ascorbinsäure und/oder Derivaten von Ascorbinsäure wie Ascorbat-2-phosphat, Proteinen der TGF-ß Superfamilie beispielsweise die TGF-β Isoformen (TGF-β1, TGF-β2, TGF-β3), Bone Morphogenetic Proteins (BMP-2, BMP-3, BMP-4, BMP-5, BMP-6, BMP-7), Growth Differentiation Factor wie beispielsweise GDF-5 und GDF-10, Insulin-like growth factor wie IGF-1, Glucocorticoiden wie Dexamethason.

5. Verfahren nach einem der vorgehenden Ansprüche, **dadurch gekennzeichnet, dass** die Zellen aus Gewebe endodermalen, ektodermalen oder mesodermalen Ursprungs isoliert werden, vorzugsweise aus muskoskeletalem Gewebe, Skelettgewebe, Knorpel, Knochen, Muskeln, glatter Muskulatur, Herzmuskel, Nieren, Nebennierenrinde, Leber, Bindegewebe, Meniskus.

6. Verfahren nach einem der vorgehenden Ansprüche **dadurch gekennzeichnet, dass** die Zellen Chondrozyten sind, beispielsweise humane Chondrozyten, vorzugsweise frisch isolierte und/oder postmitotische humane Chondrozyten.

7. Verfahren nach einem der vorgehenden Ansprüche **dadurch gekennzeichnet, dass** die isolierten Zellen durch Kultur in Monolayer vermehrt und/oder dedifferenziert werden.

8. Verfahren nach einem der vorgehenden Ansprüche **dadurch gekennzeichnet, dass** die isolierten und gegebenenfalls vermehrten und/oder dedifferenzierten Zellen zur Bildung von Sphäroiden dadurch angeregt werden, dass die Zellen in einer hydrophoben Umgebung kultiviert werden, beispielsweise auf einer Agaroseschicht.

9. Funktionales Fusionsgewebe erhältlich durch ein Verfahren nach einem einem der Ansprüche 1 bis 8.

10. Funktionales Fusionsgewebe nach Anspruch 9 **dadurch gekennzeichnet, dass** der Kollagenanteil 50 bis 70 % und der Proteoglykananteil 15 bis 45 % in Bezug auf das Trockengewicht des funktionalen Fusionsgewebes beträgt.

11. Funktionales Fusionsgewebe nach einem der Ansprüche 8 oder 9 **dadurch gekennzeichnet, dass** der Anteil des Kollagen Typ II bis zu 85 bis 98 % bezogen auf den Gesamtkollagengehalt des funktionalen Fusionsgewebes beträgt.

12. Zubereitung, insbesondere pharmazeutische Zubereitung, Arzneimittel, Transplantat bestehend aus oder enthaltend
a) funktionales Fusionsgewebe nach einem der Ansprüch 9 bis 11 und
b) gegebenenfalls weitere Zusatz- und Hilfsstoffe.

13. Zubereitung, insbesondere pharmazeutische Zubereitung, Arzneimittel, Transplantat nach Anspruch 12 zur spezifischen Anwendung zur Behandlung von rheumatischen Erkrankungen, Knorpeldefekten, Knochendefekten, insbesondere traumatischen Knorpeldefekten und/oder Knochendefekten, Läsionen, insbesondere traumatischen Läsionen, bei Knorpeldegenerationen, Knochendegeneration, Osteoarthritis, zur therapeutische Knorpelregeneration und / oder Knochenregeneration in vivo.

14. Testsystem, beispielsweise Test Kit umfassend
a) funktionales Fusionsgewebe nach einem der Ansprüche 9 bis 11 oder erhältlich durch ein Verfahren nach einem der Ansprüche 1 bis 8 und
b) gegebenenfalls weitere Hilfs- und Zusatzsstoffe.

15. Verfahren zum Testen von zu untersuchenden Substanzen
**dadurch gekennzeichnet, dass**
a) funktionales Fusionsgewebe nach einem der Ansprüche 9 bis 11 oder erhältlich durch ein Verfahren nach einem der Ansprüche 1 bis 8 oder ein Testsystem nach Anspruch 14,
b) mit einer oder mehreren zu untersuchenden Substanzen in Kontakt gebracht wird,
c) der Effekt von der/den zu untersuchenden Substanz(en) auf das funktionale Fusionsgewebe bestimmt wird.
